# EUROPEAN PATENT SPECIFICATION

(11) **EP 4 659 724 B1**
(45) Date of publication and mention of the grant of the patent: **04.11.2026**
(21) Application number: 24180305.5
(22) Date of filing: 05.06.2024
(51) Int. Cl.: A61F 13/15

(54) **AN APPARATUS AND A METHOD FOR STRETCHING A PLURALITY OF ELASTIC STRIPS IN A CROSS-MACHINE DIRECTION**
VORRICHTUNG UND VERFAHREN ZUM STRECKEN EINER VIELZAHL VON ELASTISCHEN BÄNDERN IN MASCHINENÜBERGREIFENDER RICHTUNG
APPAREIL ET PROCÉDÉ POUR ÉTIRER UNE PLURALITÉ DE BANDES ÉLASTIQUES DANS UNE DIRECTION TRANSVERSALE À LA MACHINE

(43) Date of publication of application: 10.12.2025
(73) Proprietor: Curt G. Joa, Inc., Sheboygan Falls, Wisconsin 53085-0903 (US)
(72) Inventor: MIGACZ, Mark A, Sheboygan Falls, 53085-0903 (US); JANKUSKI, Brian G, Sheboygan Falls, 53085-0903 (US); EISNER, Anthony J, Sheboygan Falls, 53085-0903 (US); FRITZ, Jeffrey W, Sheboygan Falls, 53085-0903 (US); SCHULZ, Brenden, Plymouth, 53073 (US)
(74) Representative: Potter Clarkson

(56) References cited:
- US-A1- 2012 073 729
- US-B2- 8 591 682

## Description

The present invention relates to an apparatus and a method for stretching a plurality of elastic strips in a cross-machine direction. The present invention also relates to the use of such an apparatus or method to assemble an elastic laminate and/or to manufacture an absorbent article, such as a diaper or incontinence control garment.

Absorbent articles, such as infant diapers, incontinence garments and the like, are typically formed from a combination of multiple web layers, absorbent structures, and elastic elements. The absorbent articles may include a thin flexible liquid impermeable backing sheet on which a permeable nonwoven sheet is overlayed. An absorbent pad is disposed between the two layers and the layers are adhered at their edges to form a unitary article that prevents liquid body exudates from seeping out of the edges of the absorbent article. Elastic elements, such as an elastic waist element or pockets, elastic ear panels, and elastic leg cuffs, may be provided in the absorbent article to help the absorbent article better conform to the body of the wearer around the waist, back, and legs to prevent leakage along the interface of the nonwoven layer which is in contact with the body. Elastic elements may also be provided in disposable medical products, for example as wrist cuffs in medical gowns. The following prior art documents were cited in the European Search Report: US 2012073729 A1 and US 8591682 B2.

In the manufacture of absorbent articles, elastic elements are often provided in the form of an arrangement of elastic strands (i.e. single thread-like lengths of elastic material used to impart linear elasticity) or elastic strips (i.e. broader and flatter sheet-like lengths of elastic material capable of stretching in one or in multiple directions, for imparting elasticity over a larger area) which may be provided adjacent a single nonwoven sheet or sandwiched between a top sheet and a back sheet, and which may be bonded to secure them in place. Elastic strips may be applied to a web layer in a stretched condition.

According to an aspect of the invention there is provided an apparatus for stretching a plurality of elastic strips in a cross-machine direction comprising the features recited in claim 1.

The apparatus comprises a first set of rotatable discs for stretching a first elastic strip in a cross-machine direction, and a second set of rotatable discs for stretching a second elastic strip in a cross-machine direction. A first rotatable disc of each set of rotatable discs is inclined at an angle with respect to the second rotatable disc of each set of rotatable discs. Each rotatable disc has an outer surface provided with at least one gripping element configured to receive an edge of an elastic strip at a pick-up location and hold the edge of the elastic strip as the elastic strip is advanced in the machine direction from the pick-up location to a drop-off location during operation, whereby the distance between the first rotatable disc and the second rotatable disc of each set of rotatable discs is greater at the drop-off location than at the pick-up location, thereby stretching the elastic strip in the cross-machine direction. The apparatus comprises a driving assembly comprising common power source, a common drive shaft, and a rotation-transmitting mechanism, whereby the common drive shaft and the rotation-transmitting mechanism are configured to transmit power from the common power source to both the first set of rotatable discs and the second set of rotatable discs to rotate the first rotatable disc and the second rotatable disc of each set of rotatable discs.

Since a single driving assembly is used to transmit power from the common power source to both the first set of rotatable discs and the second set of rotatable discs rather than providing a separate driving assembly for each set of rotatable discs, such an apparatus is simpler and less expensive than a conventional apparatus comprising a plurality of driving assemblies. Furthermore, such an arrangement reduces space requirements and improves access to components of the apparatus. This facilitates processes such as the threading or feeding of an elastic strip through the apparatus, configuring apparatus settings, such as speed and tension settings, cleaning and inspecting the apparatus, as well as conducting maintenance and repair work.

According to an embodiment, the rotation-transmitting mechanism comprises at least one or a combination of the following: at least one belt, at least one V-belt, at least one chain, at least one gear, at least one direct connection between the common drive shaft and a set of rotatable discs, at least one timing belt, at least one timing chain, at least one rope, at least one cable, at least one pulley, at least one sprocket, a hydraulic system, a pneumatic system.

A rotation-transmitting mechanism comprising at least one belt extends an apparatus manufacturer's design options since the common drive shaft does not need to be axially aligned with rotatable shafts on which the rotatable discs are mounted. Furthermore, a belt's inherent elasticity, flexibility and deformability allows the belt to absorb and dampen shocks and/or vibrations that occur during the operation of the apparatus and the belt drive may thereby generate a low level of noise. A belt needs no lubrication, requires minimal maintenance, and has a high tolerance for misalignment.

According to an embodiment, a first rotatable disc of at least one, or each, set of rotatable discs is configured to rotate about a first axis of rotation, and a second rotatable disc of the at least one, or each, set of rotatable discs is configured to rotate about a second axis of rotation, whereby the first axis of rotation is inclined at an angle with respect to the second axis of rotation.

According to an embodiment a first rotatable disc of at least one, or each, set of rotatable discs and a second rotatable disc of the at least one, or each, set of rotatable discs are configured to rotate about a common axis of rotation.

According to an embodiment, the apparatus comprises a support structure and at least one of the rotatable discs is slidably mounted on the support structure. One or more of the following parameters may be adjusted by sliding the at least one rotatable disc on the support structure: the minimum distance and/or the maximum distance between a first rotatable disc and a second rotatable disc of a set of rotatable discs, a spacing between the first set of rotatable discs and the second set or rotatable discs, and/or the inclination of the first rotatable disc or the first axis of rotation and/or the inclination of the second rotatable disc or the second axis of rotation.

Such a support structure enables the pitch of an elastic strip, i.e. the distance between adjacent points in a pattern of elastic, which is typically measured along the length of the elastic strip, and/or the spacing between adjacent elastic strips applied onto a carrier web to be varied as desired by sliding the at least one rotatable disc on the support structure.

According to an embodiment, the support structure is a common support structure and all of the rotatable discs of all of the sets of rotatable discs are slidably mounted on the common support structure.

According to an embodiment, the support structure or common support structure, comprises at least one rotatable disc-position indicator, such as a marking, a ruler and/or a protractor, configured to indicate a position of the at least one rotatable disc that is slidably mounted thereon.

According to an embodiment, the apparatus comprises at least one additional set of rotatable discs for stretching at least one additional elastic strip in a cross-machine direction, and the common drive shaft and rotation-transmitting mechanism are configured to transmit power from the common power source to all of the sets of rotatable discs to rotate the first rotatable disc and the second rotatable disc of each set of rotatable discs.

An apparatus according to the invention may comprise any number of sets of rotatable discs located in any desired arrangement along the cross-machine direction and/or the along machine direction, such as at least two, at least three, at least four, at least five, at least six, at least seven, at least eight, at least nine, at least ten sets of rotatable discs.

According to an embodiment, the apparatus comprises at least one supply unit configured to supply a first elastic strip to the first set of rotatable discs and a second elastic strip to the second set of rotatable discs, at least one supply unit configured to supply at least one carrier web, and at least one bonding device configured to bond the first elastic strip and the second elastic strip to the at least one carrier web.

The at least one bonding device may comprise at least one Or a combination of the following: an adhesive bonding device, an ultrasonic bonding device, a heat bonding device, a pressure bonding device, a heat and pressure bonding device. A bonding device may be located at any of the following positions: upstream of the pick-up location of a set of rotatable discs in the machine direction, at the pick-up location of a set of rotatable discs, downstream of the pick-up location of a set of rotatable discs in the machine direction, at the drop-off location of a set of rotatable discs, downstream of the drop-off location of a set of rotatable discs in the machine direction.

According to another aspect of the invention there is provided a method for stretching a plurality of elastic strips in a cross-machine direction, such as at least two elastic strips, at least three elastic strips, at least four elastic strips, at least five elastic strips, at least six elastic strips, at least seven elastic strips, at least eight elastic strips, at least nine elastic strips, or at least ten elastic strips.

The method comprises the features recited in the independent method claim.

The method comprises supplying a first elastic strip to a first set of rotatable discs for stretching a first elastic strip in a cross-machine direction and supplying a second elastic strip to a second set of rotatable discs for stretching a second elastic strip in a cross-machine direction. The method also comprises rotating a first rotatable disc of each set of, and rotating a second rotatable disc of each set of rotatable discs, whereby the first rotatable disc is inclined at an angle with respect to the second rotatable disc.

The method also comprises receiving an edge of an elastic strip on an outer surface of a first rotatable disc of each set of rotatable discs and an edge of the elastic strip on an outer surface of a second rotatable disc of each set of rotatable discs at a pick-up location, using at least one gripping element, and advancing the elastic strip in the machine direction from the pick-up location to a drop-off location, whereby the distance between the first rotatable disc and the second rotatable disc of each set of rotatable discs is greater at the drop-off location than at the pick-up location, thereby stretching the elastic strip in the cross-machine direction.

The method further comprises rotating the first rotatable disc and the second rotatable disc of each set of rotatable discs by transmitting power from a common power source to both the first set of rotatable discs and the second set of rotatable discs via a common drive shaft and a rotation-transmitting mechanism.

According to an embodiment, the rotation-transmitting mechanism may comprise at least one, or a combination, of the following: at least one belt, at least one V-belt, at least one chain, at least one gear, at least one direct connection between the common drive shaft and a set of rotatable discs, at least one timing belt, at least one timing chain, at least one rope, at least one cable, at least one pulley, at least one sprocket, a hydraulic system, a pneumatic system.

According to an embodiment, the method comprises rotating a first rotatable disc of at least one, or each, set of rotatable discs about a first axis of rotation and rotating a second rotatable disc of the at least one, or each, set of rotatable discs about a second axis of rotation, whereby the first axis of rotation is inclined at an angle with respect to the second axis of rotation.

According to an embodiment, the method comprises rotating a first rotatable disc of at least one, or each, set of rotatable discs and a second rotatable disc of the at least one, or each, set of rotatable discs about a common axis of rotation.

According to an embodiment, the method comprises slidably mounting at least one rotatable disc of the at least one set of rotatable discs on a support structure, and adjusting at least one of the following parameters by sliding the at least one rotatable disc on the support structure: the minimum distance and/or the maximum distance between the first rotatable disc and the second rotatable disc of a set of rotatable discs, a spacing between the first set of rotatable discs and the second set of rotatable discs, and/or the inclination of the first rotatable disc or the first axis of rotation and/or the inclination of the second rotatable disc or the second axis of rotation.

According to an embodiment, the method comprises providing a common support structure and slidably mounting all of the rotatable discs of all of the sets of rotatable discs on the common support structure.

According to an embodiment, the method comprises providing the support structure, or the common support structure, with at least one rotatable disc-position indicator to indicate a position of the at least one rotatable disc mounted on the support structure.

According to an embodiment, the method comprises: supplying a first elastic strip to the first set of rotatable discs, supplying a second elastic strip to the second set of rotatable discs, supplying at least one carrier web, and bonding a stretched first elastic strip and a stretched second elastic strip to the at least one carrier web. A carrier web may be a nonwoven layer or any other component of an elastic laminate or absorbent article.

According to an embodiment, the method comprises supplying two carrier webs and bonding a stretched first elastic strip and a stretched second elastic strip between the two carrier webs.

In a further aspect of the invention there is provided the use of an apparatus according to any of the embodiments described herein, or a method according to any of the embodiments described herein to assemble an elastic laminate and/or to manufacture an absorbent article, such as a disposable absorbent article.

All embodiments of the invention and particular features mentioned herein may be taken in isolation or in combination with any other embodiments and/or particular features mentioned herein (hence describing more particular embodiments and particular features as disclosed herein) without departing from the disclosure of the invention.

Unless otherwise indicated, all technical and scientific terms used herein have the same meaning as commonly understood by one of ordinary skill in the art to which this invention pertains.

As used herein, the term "elastic" is intended to mean the ability of a material to stretch by at least 5%, or at least 10%, or at least 20%, or at least 30%, or at least 40%, or at least 50% of its original length without rupture and return to its original length. This elasticity is not limited to the ability of a material to stretch along its length. A material may stretch by these amounts in any direction, such as along its width or in a diagonal direction in which the material simultaneously stretches in both its length and width directions simultaneously.

As used herein, the term "elastic strip" is intended to mean a means a sheet-like material wherein the length and width of the material far exceed the thickness of the material. For example, the length and width of the material are at least 10 times or at least 20 times, or at least 50 times, or at least 100 times, or at least 200 times, or at least 500 times its thickness. An elastic strip may have a width of at least 1 cm measured in a cross-machine direction in a non-stretched condition, or at least 2 cm, or at least 3cm, or at least 4 cm, or at least 5 cm, or at least 6 cm, or at least 7 cm, or at least 8 cm, or at least 9 cm, or at least 10m. The term does not include elastic strands, i.e. single thread-like lengths of elastic material used to impart linear elasticity, which have a width of less than 1 cm measured in a cross-machine direction in a non-stretched condition. An elastic strip may be supplied in the form of a continuous elastic strip, or a discontinuous elastic strip, i.e. an elastic strip supplied as a series of discrete elastic strips.

The term "an edge of an elastic strip" is intended to mean not only an outer boundary of the elastic strip in a stretched or unstretched condition in a cross-machine direction, but also an area of the stretched or unstretched elastic strip within a distance from the outer boundary of the stretched or unstretched elastic strip in a cross-machine direction. The distance may correspond to up to 30%, or up to 20%, or up to 10%, or up to 5% of the total extension of the stretched or unstretched elastic strip in the cross-machine direction.

As used herein, the term "rotatable disc" is intended to mean any mechanical component that is capable of turning or rotating around its axis of rotation. A rotatable disc may be of any suitable shape and/or of any suitable size and not necessarily a flat circular object. A rotatable disc may comprise a circular disc, a conical disc, a ring, a segmented ring comprising a plurality of ring segments arranged around a central axis, a portion of a sphere. A rotatable disc may have a regular or an irregular shape.

Likewise, terms such as "periphery", "radial direction" and "axial direction" are not intended to imply that a rotatable disc is necessarily a circular object, but are intended to also include the corresponding term when a "rotatable disc" is not a circular object, such as "perimeter", "a direction outwards from the rotational axis of the "rotatable disc"" and "a direction parallel to the axis of rotation of the "rotatable disc"" respectively.

A "set" of rotatable discs may consist of, or comprise a pair of rotatable discs, i.e. the first and second rotatable discs. The rotatable discs may be of any size and/or any shape. In embodiments of the apparatus and method comprising a plurality of sets of rotatable discs, the rotatable discs of a first set of rotatable discs may have a different size and/or a different shape than the rotatable discs of a second set of rotatable discs. A first rotatable disc of a set of rotatable discs may have a different size and/or a different shape than the second rotatable disc of a set of rotatable discs.

The term "machine direction" (MD) is used herein to refer to the direction of material flow through a process.

The term "cross-machine direction" (CD) is used herein to refer to a direction that is perpendicular, or substantially perpendicular, to the machine direction.

As used herein, the term "minimum distance" is intended to mean the smallest length of space between the first rotatable disc and the second rotatable disc of a set of rotatable discs in the cross-machine direction measured between the innermost parts of the outer surfaces of the set of rotatable discs in the cross-machine direction. In the apparatus according to the invention, the edges of an elastic strip may be picked up at the location at which the first rotatable disc and the second rotatable disc of a set of rotatable discs are at a minimum distance from each other, or at a location upstream or downstream from that location. The elastic strip is then advanced in the machine direction towards the location at which the first rotatable disc and the second rotatable disc of a set of rotatable discs are at a maximum distance from each other, or at a location upstream or downstream from that location, thereby stretching the elastic strip. The stretched elastic strip may then be taken off the set of rotatable discs, by transferring the stretched elastic strip to a rotating anvil or to a transfer roll for example. The minimum distance may be up to 10 mm, up to 20 mm, up to 30 mm, up to 40 mm, up to 50 mm, up to 60 mm, up to 70 mm, up to 80 mm, up to 90 mm, up to 100 mm, up to 150 mm, up to 200 mm, up to 300 mm, or more.

As used herein, the term "maximum distance" is intended to mean the greatest length of space between the first rotatable disc and the second rotatable disc of a set of rotatable discs space measured between the innermost parts of the outer surfaces the set of rotatable discs in the cross-machine direction.

The term "common power source" is intended to mean a power supply configured to solely provide the power to cause the common drive shaft to rotate. A common power source may be configured to convert one form of energy into another form, such as a power source configured to convert electrical energy into rotational motion to cause the common drive shaft to rotate. A common power source may comprise one, or a combination of the following: an electric motor or an electric drive, a servomotor, a hydraulic drive, a pneumatic drive, a mechanical drive, a generator, a battery, and/or an internal combustion engine. A common power source may for example comprise two motors whose output shafts are mechanically coupled using gears, belts or other couplings to synchronize the rotation of the two output shafts to rotate the common drive shaft.

The term "at least one gripping element" is intended to mean at least one element configured to receive an edge of an elastic strip and hold it while the elastic strand is advanced from the pick-up location to the drop-off location of a set of rotatable discs in the machine direction during operation. The at least one gripping element may comprise at least one radially protruding element, such as at least one pin and/or at least one aperture that is fluidly connected to a vacuum pressure source during the operation of the apparatus. The vacuum pressure source may be configured to draw an edge of an elastic strip toward the at least one aperture to receive and hold the edge of the elastic strip on the outer surface of the rotatable disc. The at least one gripping element may extend along at least one part of an outer surface of at least one rotatable disc of a set of rotatable discs, or around the entire outer surface of at least one rotatable disc of a set of rotatable discs. According to an embodiment of the invention, the apparatus may comprise equipment to facilitate the release of an edge of a stretched elastic strip from the at least one gripping element at the drop-off location.

The term "disposable absorbent article" is intended to mean an absorbent article that is not intended to be laundered or otherwise restored and re-used as an absorbent article. Instead, a disposable absorbent article is intended to be discarded after a single use and preferably recycled.

As used herein, the term "comprises" will take its usual meaning in the art, namely indicating that the component includes but is not limited to the relevant features (i.e., including, among other things). As such, the term "comprises" will include references to the component consisting essentially of the relevant feature(s). For the avoidance of doubt, the term "comprises" will also include references to the component "consisting essentially of" (and in particular "consisting of") the relevant feature(s).

The invention is illustrated by way of the following examples, which are not intended to be limiting on the general scope of the invention.
**Figure 1****:** schematically shows a side view of an apparatus according to an embodiment of the invention operating to assemble an elastic laminate,
**Figure 2****:** schematically shows a plan view of part of an apparatus according to an embodiment of the invention,
**Figure 3****:** schematically shows a driving assembly of an apparatus according to an embodiment of the invention,
**Figure 4****:** schematically shows a perspective view of a support structure of an apparatus according to an embodiment of the invention,
**Figure 5****:** schematically shows a front elevation view of the support structure of Figure 4 according to an embodiment of the invention,
**Figure 6****:** schematically shows a perspective view of a support structure of an apparatus according to an embodiment of the invention,
**Figure 7****:** schematically shows a front elevation view of the support structure of Figure 6 according to an embodiment of the invention, and
**Figure 8****:** is a flow chart showing the steps of a method according to an embodiment of the invention.

The drawings have not necessarily been drawn to scale and the dimensions of certain features may have been exaggerated for the sake of clarity.

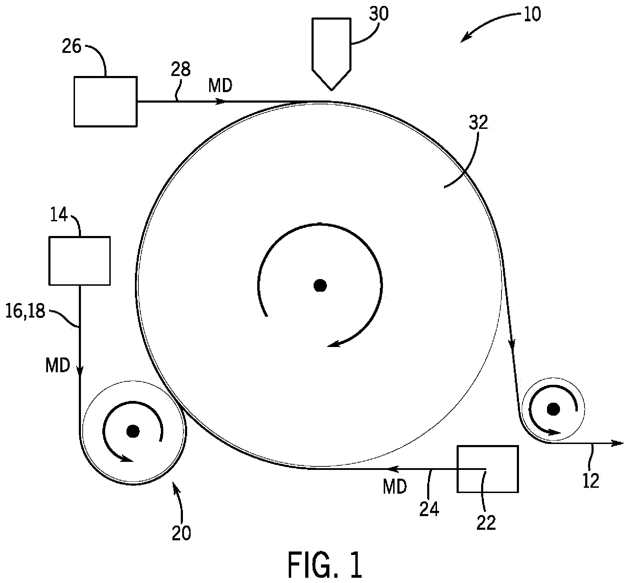

Figure 1 schematically shows a side view of an apparatus 10 according to an embodiment of the invention operating to assemble an elastic laminate 12 comprising a plurality of elastic strips 16, 18. The apparatus 10 may be used to manufacture an absorbent article, such as a disposable absorbent article, or to manufacture an intermediary composite material for later use in the manufacture of an absorbent article.

The apparatus 10 comprises a supply unit 22 configured to supply a first carrier web 24, to an outer surface of a rotating anvil 32, i.e. a cylindrical or drum-like component that is configured to rotate continuously during the manufacturing process. The first carrier web 24 may be a liquid-permeable or liquid impermeable nonwoven layer, or any other component of an absorbent article.

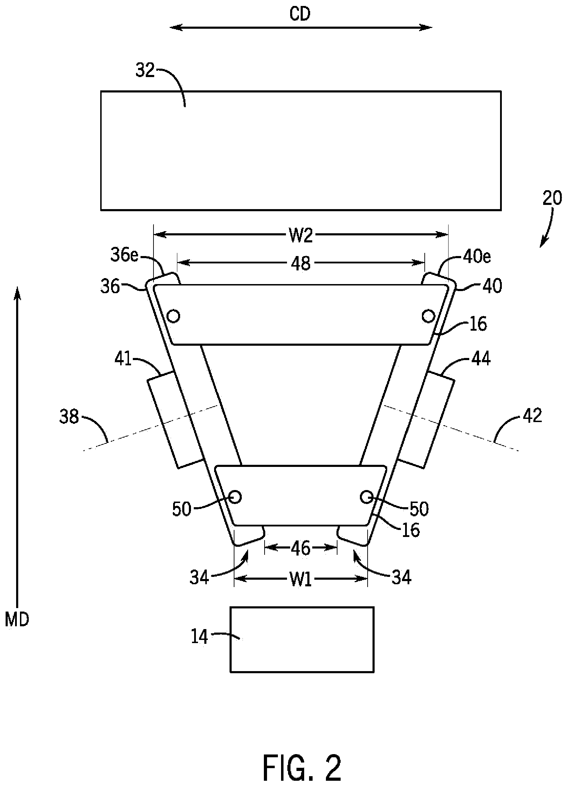

The apparatus 10 also comprises a supply unit 14 configured to supply a first elastic strip 16 to a first set of rotatable discs of a spreader mechanism 20 for stretching the first elastic strip 16 in a cross-machine direction and to supply a second elastic strip 18 to a second set of rotatable discs of a spreader mechanism 20 for stretching the second elastic strip 18 in a cross-machine direction. According to alternative embodiments supply unit 14 may include individual material feed rolls that supply the first and second elastic strips 16, 18 or a single material feed with a downstream slitter that divides the width of the single material roll into separate continuous strips. The spreader mechanism will be described in more detail with reference to Figure 2.

The apparatus 10 illustrated in Figure 1 further comprises a supply unit 26 configured to supply a second carrier web 28 to the layers on the outer surface of the rotating anvil 32. The second carrier web 28 may be a liquid-permeable or liquid impermeable nonwoven layer, or any other component of an absorbent article.

The apparatus 10 also comprises a bonding device 30, such as an ultrasonic horn, configured to bond the stretched first elastic strip 16 and stretched second elastic strip 18 between the first carrier web 24 and the second carrier web 28.

Any or all of supply unit 14, supply unit 22, and supply unit 26 may be provided with means to guide or oscillate the center line of the supplied webs or strips relative to the cross-machine direction (CD).

During the operation of the apparatus 10, the first carrier web 24 advances in the machine direction MD towards and along the outer surface of a rotating anvil 32. The spreader mechanism 20 stretches the elastic strips 16, 18 in a cross-machine direction. The stretched elastic strips 16, 18 are then positioned into contact with the first carrier web 24 on the outer surface of a rotating anvil 32 and maintained in a stretched condition. The second carrier web 28 is advanced and positioned into contact with and attached to the layers on the outer surface of the rotating anvil 32.

As the anvil 32 rotates, the first carrier web 24, the stretched elastic strips 16,18 and the second carrier web 28 are advanced between the outer surface of the anvil 32 and the bonding device 30 to bond the first carrier web 24, the stretched elastic strips 16, 18 and the second carrier web 28 together to form an elastic laminate 12. The elastic laminate 12 may then be advanced from the anvil 32 to one or more additional absorbent article assembly processes or stored until needed for production. In a relaxed state, the stretched elastic strips 16, 18 will contract in the cross-machine direction.

Continuous operation of such an apparatus 10 permits the high-speed assembly of elastic laminates 12 comprising a plurality of elastic strips 16, 18 which in turn facilitates the high-speed manufacture of absorbent articles, such as diapers or incontinence control garments.

Figure 2 schematically shows a plan view of a set of rotatable discs 34 of a spreader mechanism 20 for stretching a first elastic strip 16 in a cross-machine direction CD. The spreader mechanism 20 also comprises a second set of such rotatable discs (not shown) for stretching a second elastic strip in a cross-machine direction CD.

In the illustrated embodiment, a first elastic strip 16 is supplied to the spreader mechanism 20 in the form of a non-continuous first elastic strip 16, i.e. in a series of discrete elastic strips 16. Alternatively, the elastic strip 16 is supplied to the spreader mechanism 20 in the form of a continuous elastic strip 16.

According to an embodiment, the apparatus 10 according to the present invention may comprise at least one cutting device, such as at least one rotating or oscillating cutting element (not shown), which is configured to cut at least one continuous elastic strip into a plurality of non-continuous elastic strips.

The outer surfaces 36e and 40e of the first rotatable disc 36 and the second rotatable disc 40 are provided with a plurality of gripping elements 50, such as a plurality of apertures that are fluidly connected to a vacuum pressure source and/or structural teeth coupled to or integrated within the outer surfaces 36e and 40e of the first and second rotatable discs 36, 40and/or one or more projections coupled to or integrated within the outer surfaces 36e and 40e of the first and second rotatable discs 36,40. The gripping elements 50 are configured to receive and hold an edge of the first elastic strip 16 as it passes over the outer surfaces 36e, 40e of the rotating discs 36, 40.

A first rotatable disc 36 of the set of rotatable discs 34 is configured to rotate about a first axis of rotation 38, and a second rotatable disc 40 of the set of rotatable discs 34 is configured to rotate about a second axis of rotation 42. The first rotatable disc 36 may comprise a hub or be mounted on a first shaft 41. Likewise, the second rotatable disc 36 may comprise a hub or be mounted on a second shaft 44. The first axis of rotation 38 is inclined at an angle with respect to the second axis of rotation 42. In an alternative embodiment, first and second discs 36, 40 are oriented such that they are inclined at an angle relative to one another, as shown in Figure 2, but configured to rotate about a common axis of rotation via a constant velocity (CV) drive (not shown).

This angled orientation of the first rotatable disc 36 and the second rotatable disc 40 creates a pulling or stretching force on an elastic strip 16 whose edges are held at the outer surfaces 36e and 40e of the first rotatable disc 36 and the second rotatable disc 40 by the plurality of gripping elements 50 as the elastic strip 16 passes over the rotating discs 36, 40. The stretching of the elastic strip 16 in the cross-machine direction CD may thereby be precisely controlled and the use of such a spreader mechanism 20 in the manufacture of absorbent articles helps to provide the flexibility for the absorbent articles to fit comfortably and effectively, ensuring consistent and reliable performance.

It should be noted that both the first rotatable disc 36 and the second rotatable disc 40 of a set of rotatable discs 34 do not necessarily have to be positioned at an angle to the machine direction MD. According to an embodiment, either the first rotatable disc 36 or the second rotatable disc 44 of a set of rotatable discs 34 may be configured to rotate about an axis of rotation that extends along the cross-machine direction CD as long as the first axis of rotation 38 about which the first rotatable disc 36 rotates is inclined at an angle with respect to the second axis of rotation 42 about which the second rotatable disc 40 rotates.

Furthermore, a pick-up location and a drop-off location need not necessarily correspond to the exact locations where the first rotatable disc 36 and the second rotatable disc 40 of a set of rotatable discs 34 are at a minimum distance 46 and a maximum distance 48 respectively. An elastic strip 16, 18 may be picked up by a set of rotatable discs 34 at any location downstream of the minimum distance 46, and it may be taken off the set of rotatable discs 34 at any location upstream of the maximum distance 48.

An elastic strip 16 having a width, W1 in an unstretched condition at the pick-up location is thereby stretched in the cross-machine direction CD to have a width, W2 in a stretched condition at the drop-off location as the rotating discs 36, 40 rotate.

The stretched elastic strip 16 may be transferred to the outer surface of a rotating anvil 32 directly, or indirectly via one or more transfer rolls.

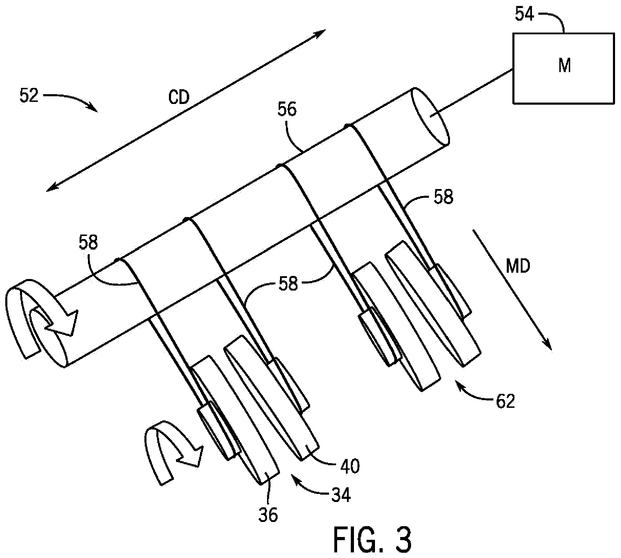

Figure 3 schematically shows a driving assembly 52 of an apparatus 10 according to an embodiment of the invention. The driving assembly 52 comprises a common power source 54, a common drive shaft 56, and a rotation-transmitting mechanism illustrated in the form of a plurality of belts 58. Each belt 58 is connected between the common drive shaft 56 and a set of rotatable discs 34, 62 of the spreader mechanism 20.

The common power source 54 may be an electric motor. The common drive shaft 56 may be a solid or tubular shaft that is configured to rotate about its longitudinal axis.

A belt 58 may comprise a continuous loop of any suitable material, such as rubber, silicone, polyvinyl chloride (PVC), a tensile cord such as fiberglass, Kevlar, carbon fiber, polyester, steel, or Nylon, an elastomer, or a synthetic polymer. Each belt 58 is connected between the common drive shaft 56 and a rotatable shaft 41, 44 on which a rotatable disc 36, 40 is mounted. Each belt 58 wraps around these shafts creating friction between the belt 58 and the shaft surfaces. When the common power source 54 causes the common drive shaft 56 to rotate, rotational motion is transferred to the belts 58, which in turn cause the driven shafts to rotate.

According to embodiments, a belt 58 may comprise a straight belt or comprise a twist belt, including a half-twist, provided by twisting the ends of the belt 58 before joining its ends to form a Möbius strip so that wear can be evenly distributed on both sides of the belt 58.

According to an embodiment, a driving assembly 52 may also comprise at least one pulley (not shown), such as at least one-stepped pulley, and/or at least one toothed pulley, and/or at least one tapered pulley. For example, a belt 58 may be used to connect a pulley that is attached to the common drive shaft 56 (the driving shaft) to a pulley attached to a shaft 41, 44 on which a rotatable disc 36, 40 is mounted (a driven shaft). The belt 58 wraps around these pulleys creating friction between the belt 58 and the pulley surfaces. When the common power source 54 causes the common drive shaft 56 to rotate, the pulleys that are attached to the common drive shaft 56 rotate and transfer rotational motion to the belts 58, which in turn cause the pulleys on the driven shafts to rotate, thereby transferring rotational motion to the rotatable discs 36, 40. The driving assembly 52 may also include one or more sprockets (not shown) that engage with the belt 58 to transfer rotational motion of the common drive shaft 56 to the rotatable discs 36, 40.

In such a two-pulley drive assembly 52, a belt 58 can either drive the pulleys normally in one direction, or the belt 58 may be crossed, so that the direction of a driven shaft is reversed. Such a drive assembly 52 can also be used to change the speed of rotation, either up or down, by using different sized pulleys.

A belt 58 may have any suitable thickness and/or any suitable cross-sectional profile, such as a rectangular, circular, or V-shaped cross-sectional profile and/or it may be a timing belt having a toothed profile. A belt 58 may be reinforced, for example by embedding fibres, such as Kevlar fibres in the belt material.

The driving assembly 52 may be configured to rotate at least one set of rotatable discs 34, 62 at a constant speed, or a non-constant speed.

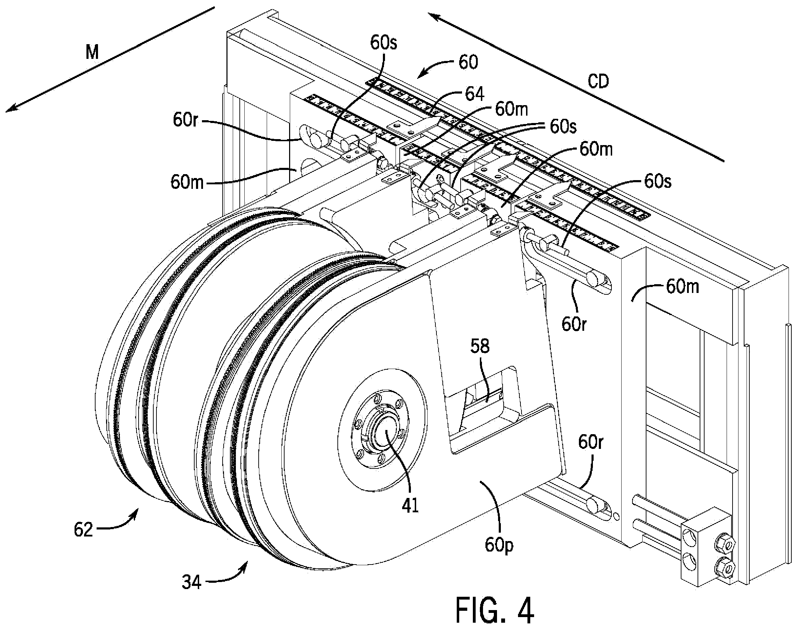

Figure 4 schematically shows a perspective view of a support structure 60 of an apparatus 10 according to an embodiment of the invention in which a first set of rotatable discs 34 is mounted at a distance from a second set of discs 62 in the cross-machine direction CD. It should be noted that a second set of discs 62 may be mounted in any desired location with respect to a first set of rotatable discs 34, such as upstream or downstream of the first set of rotatable discs 34 in the machine direction.

According to an embodiment of the invention a support structure 60 may be configured to allow at least one rotatable disc 36, 40 to be slidably moved in at least one of the following ways: along the machine direction (MD), along a cross-machine direction (CD), in a direction perpendicular to both machine direction (MD) and the cross-machine direction (CD).

In the illustrated embodiment, each disc of a first set of rotatable discs 34 and a second set of rotatable discs 62 is slidably mounted on the support structure 60.

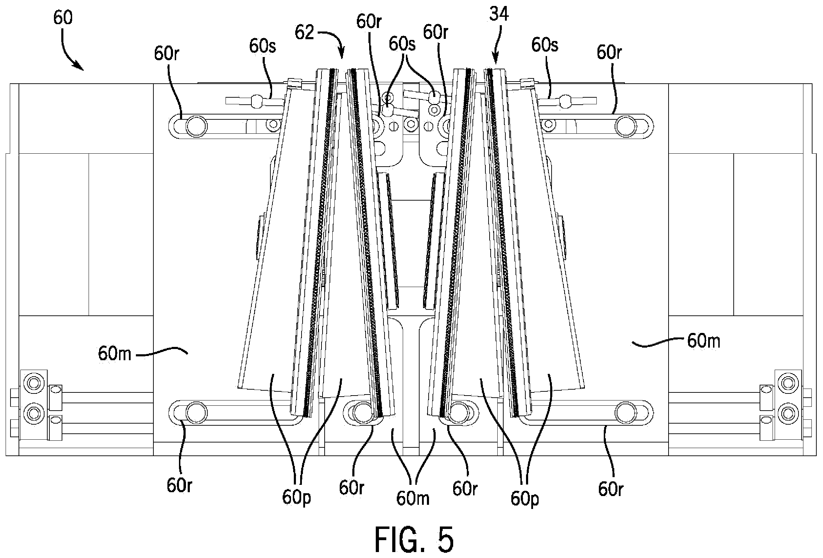

With continued reference to Figure 4 and also reference to Figure 5 as appropriate, each rotatable disc of each set of rotatable discs 34, 62 is connected to a side panel 60p the bottom and top ends of which are mounted on the rails 60r of a mounting plate 60m. The side panel 60p may function as the vacuum manifold for supplying vacuum to the rotatable discs 34, 62 in some embodiments, or be separate therefrom. The position of each rotatable disc of each set of rotatable discs 34, 62 can be adjusted by sliding respective mounting plate 60m coupled to the respective top and the bottom ends of the side panel 60p along the rails 60r in the cross-machine direction. Likewise, the inclination of each rotatable disc of each set of rotatable discs 34, 62 can be adjusted by sliding the top end of the side panel 60p along its respective angled rail 60s.

At least one of the following parameters may be adjusted: the minimum distance 46 and/or the maximum distance 48 between the first rotatable disc 36 and the second rotatable disc 40 of a set of rotatable discs 34, a spacing between the first set of rotatable discs 34 and the second set or rotatable discs 62, whereby the spacing may be measured along the cross-machine direction CD between corresponding locations on each set of rotatable discs 34, 62, an inclination of an axis of rotation 38, 42. Once a desired parameter has been selected, each rotatable disc may be locked in place on the mounting structure 60.

The support structure 60 may comprise at least one rotatable disc-position indicators 64, in the form of at least one ruler and/or at least one protractor, configured to indicate a position and/or inclination of each rotatable disc that is slidably mounted thereon.

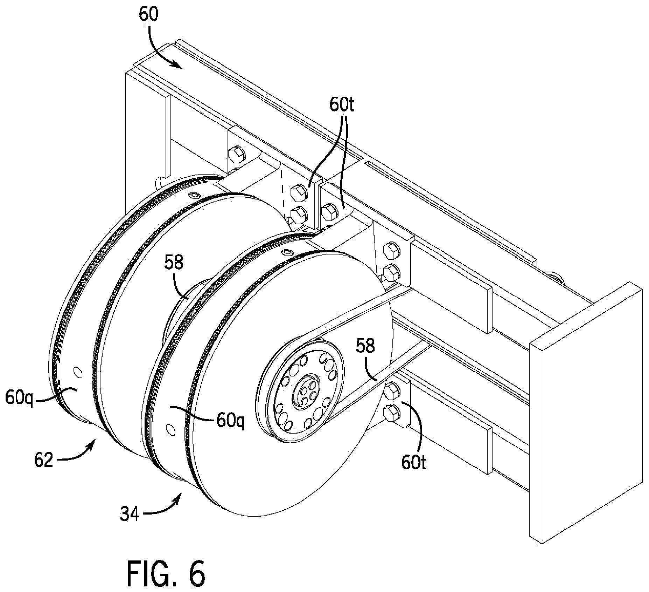

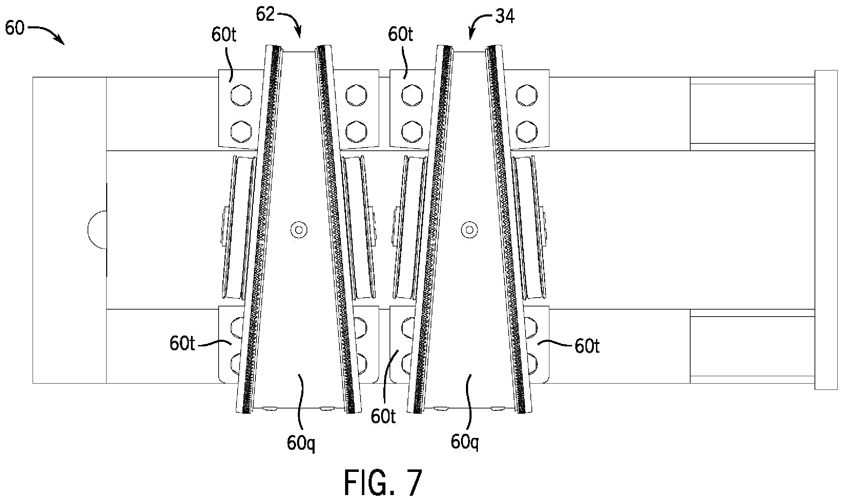

Alternatively, one, some, or all of the rotatable discs 36, 40 of one or more sets of rotatable discs 34, 62 of an apparatus 10 may be mounted in a fixed, i.e. non-adjustable, position via one or more mounting brackets 60t, as shown in the embodiment illustrated in Figures 6 and 7. In such an embodiment the relative inclination of the two rotatable discs of a set of rotatable discs 34, 62 may be fixed (i.e., not adjustable). A vacuum may be supplied to the rotatable discs of each of the one or more sets of rotatable discs 34, 62. via a common vacuum manifold 60q that is coupled to the mounting bracket(s) 60t.

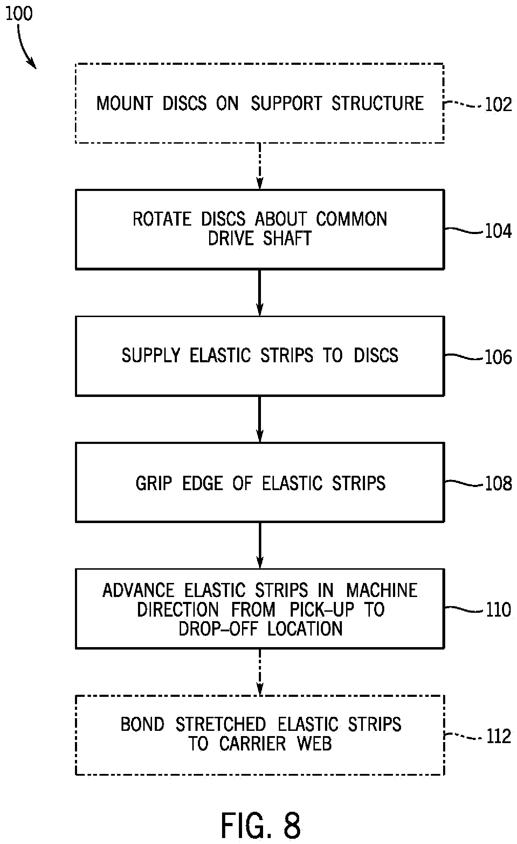

Figure 8 is a flow chart showing a method according to an embodiment of the invention. Optional steps are shown using dashed lines.

The method comprises mounting at least two sets of rotatable discs 34, 62 as described herein on a support structure 60. Preferably, at least one rotatable disc is slidably mounted on a support structure 60 that allows the position of each rotatable disc to be adjusted. The method comprises rotating the discs 36, 40 of each set of rotatable discs 34, 62 by transmitting power from a common power source 54 to all of the sets of rotatable discs 34, 62 via a common drive shaft 56 and a rotation-transmitting mechanism 58, such as at least one belt, optionally using pulleys to control the rotational speed of each rotatable disc 36, 40.

The method also comprises supplying an elastic strip 16, 18 to each set of rotatable discs 34, 62 and using gripping elements 50 to receive and hold an edge of each elastic strip 16, 18 on an outer surface 36e of a first rotatable disc 36e of each set of rotatable discs 34, 62, and an opposing edge of the elastic strip 16, 18 on an outer surface 40e of a second rotatable disc 40 of each set of rotatable discs 34, 62 at a pick-up location.

The method further comprises advancing each elastic strip 16, 18 in the machine direction MD from the pick-up location to a drop-off location, whereby the distance between the first rotatable disc 36 and the second rotatable disc 40 of each set of rotatable discs 34, 62 is greater at the drop-off location than at the pick-up location, thereby stretching the elastic strips 16, 18 in the cross-machine direction CD.

Optionally, the method comprises bonding the stretched elastic strips 16, 18 to at least one carrier web 24, 28 to produce an elastic laminate 12.

Preferences and options for a given aspect, feature or parameter of the invention should, unless the context indicates otherwise, be regarded as having been disclosed in combination with any and all preferences and options for all other aspects, features and parameters of the invention. For example, preferences and options disclosed in relation to an apparatus according to the present invention should be regarded as having been disclosed in combination with the method according to the present invention, and vice versa.

## Claims

1. An apparatus (10) for stretching a plurality of elastic strips (16, 18) in a cross-machine direction (CD), the apparatus (10) comprising:
- a first set of rotatable discs (34) for stretching a first elastic strip (16) in a cross-machine direction (CD), and
- a second set of rotatable discs (62) for stretching a second elastic strip (18) in a cross-machine direction (CD), whereby:
- a first rotatable disc (36) of each set of rotatable discs (34, 62) is inclined at an angle with respect to a second rotatable disc (40) of each set of rotatable discs (34, 62), and
∘ each rotatable disc (36, 40) has an outer surface (36e, 40e) provided with at least one gripping element (50) configured to receive an edge of an elastic strip (16, 18) at a pick-up location and hold the edge of the elastic strip (16, 18) as the elastic strip (16,18) is advanced in the machine direction (MD) from the pick-up location to a drop-off location during operation, whereby the distance between the first rotatable disc (36) and the second rotatable disc (40) of each set of rotatable discs (34, 62) is greater at the drop-off location than at the pick-up location, thereby stretching the elastic strip (16,18) in the cross-machine direction (CD),
wherein the apparatus (10) comprises:
- a common power source (54),
- a common drive shaft (56), and
- a rotation-transmitting mechanism (58),
whereby the common drive shaft (56) and the rotation-transmitting mechanism (58) are configured to transmit power from the common power source (54) to both the first set of rotatable discs (34) and the second set of rotatable discs (62) to rotate the first rotatable disc (36) and the second rotatable disc (40) of each set of rotatable discs (34, 62).

2. The apparatus (10) according to claim 1, wherein the rotation-transmitting mechanism (58) comprises at least one or a combination of the following: at least one belt, at least one V-belt, at least one chain, at least one gear, at least one direct connection between the common drive shaft (56) and a set of rotatable discs (34, 62), at least one timing belt, at least one timing chain, at least one rope, at least one cable, at least one pulley, at least one sprocket, a hydraulic system, a pneumatic system.

3. The apparatus (10) according to claim 1 or claim 2, wherein a first rotatable disc (36) of at least one set of rotatable discs (34, 62) is configured to rotate about a first axis of rotation (38), and a second rotatable disc (40) of the at least one set of rotatable discs (34, 62) is configured to rotate about a second axis of rotation (42), whereby the first axis of rotation (38) is inclined at an angle with respect to the second axis of rotation (42)

4. The apparatus (10) according to any of claims 1-3, wherein the apparatus (10) comprises a support structure (60) and at least one of the rotatable discs (36, 40) is slidably mounted on the support structure (60).

5. The apparatus (10) according to claim4, wherein the support structure (60) is configured to allow at least one of the following parameters to be adjusted by sliding the at least one rotatable disc (36, 40) on the support structure (60):
- the minimum distance (46) and/or the maximum distance (48) between the first rotatable disc (36) and the second rotatable disc (40) of a set of rotatable discs (34, 62),
- a spacing between the first set of rotatable discs (34) and the second set of rotatable discs (62),
- the inclination of the first rotatable disc (36) or the first axis of rotation (38),
- the inclination of the second rotatable disc (40) or the second axis of rotation (42).

6. The apparatus (10) according to claim 4 or claim 5, wherein the support structure (60) is a common support structure, and all of the rotatable discs (36, 40) are slidably mounted on the common support structure (60).

7. The apparatus (10) according to any of claims 3-6, wherein the support structure (60) comprises at least one rotatable disc-position indicator (64) configured to indicate a position of the at least one rotatable disc (36, 40) that is slidably mounted thereon.

8. The apparatus (10) according to any preceding claim, wherein the apparatus (10) comprises at least one additional set of rotatable discs for stretching at least one additional elastic strip in a cross-machine direction (CD), and the common drive shaft (56) and the rotation-transmitting mechanism are configured to transmit power from the common power source (54) to all of the sets of rotatable discs to rotate the first rotatable disc (36) and the second rotatable disc (40) of each set of rotatable discs.

9. The apparatus (10) according to any preceding claim, wherein the apparatus (10) comprises:
- at least one supply unit (14) configured to supply a first elastic strip (16) to the first set of rotatable discs (34) and a second elastic strip (18) to the second set of rotatable discs (62),
- at least one supply unit (22, 26) configured to supply at least one carrier web (24, 28), and
- at least one bonding device (30) configured to bond a stretched first elastic strip (16) and a stretched second elastic strip (18) to the at least one carrier web (24, 28).

10. A method for stretching a plurality of elastic strips (16,18) in a cross-machine direction (CD), the method comprising:
- supplying a first elastic strip (16) to a first set of rotatable discs (34) for stretching a first elastic strip (16) in a cross-machine direction (CD),
- supplying a second elastic strip (18) to a second set of rotatable discs (62) for stretching a second elastic strip (18) in a cross-machine direction (CD),
- rotating a first rotatable disc (36) of each set of rotatable discs (34, 62), and
- rotating a second rotatable disc (40) of each set of rotatable discs (34, 62), whereby the first rotatable disc (36) is inclined at an angle with respect to the second rotatable disc, and
- receiving an edge of an elastic strip (16, 18) on an outer surface (36e, 40e) of a first rotatable disc (36) of each set of rotatable discs and an edge of the elastic strip (16, 18) on an outer surface (36e, 40e) of a second rotatable disc (40) of each set of rotatable discs at a pick-up location,
- holding the elastic strip (16, 18) on the outer surface (36e, 40e) of each rotatable disc (36, 40) using at least one gripping element (50), and
- advancing the elastic strip (16, 18) in the machine direction (MD) from the pick-up location to a drop-off location, whereby the distance between the first rotatable disc (36) and the second rotatable disc (40) of each set of rotatable discs (34, 62) is greater at the drop-off location than at the pick-up location, thereby stretching the elastic strip (16, 18) in the cross-machine direction (CD),
wherein the method comprises:
- rotating the first rotatable disc (36) and the second rotatable disc (40) of each set of rotatable discs (34, 62) by transmitting power from a common power source (54) to both the first set of rotatable discs (34) and the second set of rotatable discs (62) via a common drive shaft (56) and a rotation-transmitting mechanism (58),

11. The method according to claim 10, wherein the method comprises providing a rotation-transmitting mechanism (58) that comprises at least one or a combination of the following: at least one belt, at least one V-belt, at least one chain, at least one gear, at least one direct connection between the common power source (54) and common drive shaft (56), at least one timing belt, at least one timing chain, at least one rope, at least one cable, at least one pulley, at least one sprocket, a hydraulic system, a pneumatic system.

12. The method according to claim 10 or claim 11, wherein the method comprises slidably mounting at least one rotatable disc (36, 40) of the at least one set of rotatable discs (34, 62) on a support structure (60), and adjusting at least one of the following parameters by sliding the at least one rotatable disc (36, 40) on the support structure (60):
- the minimum distance (46) and/or the maximum distance (48) between the first rotatable disc (36) and the second rotatable disc (40) of a set of rotatable discs (34, 62),
- a spacing between the first set of rotatable discs (34) and the second set of rotatable discs (62),
- the inclination of the first rotatable disc (36) or the first axis of rotation (38),
- the inclination of the second rotatable disc (40) or the second axis of rotation (42).

13. The method according to claim 12, wherein the method comprises providing a common support structure (60) and slidably mounting all of the rotatable discs (36, 40) of all of the sets of rotatable discs (34, 62) on the common support structure (60).

14. The method according to any of claims 10-13, wherein the method comprises:
- supplying a first elastic strip (16) to the first set of rotatable discs (34),
- supplying a second elastic strip (18) to the second set of rotatable discs (62),
- supplying at least one carrier web (24, 28), and
- bonding the first elastic strip (16) and the second elastic strip (18) to the at least one carrier web (24, 28).

15. Use of an apparatus (10) according to any of claims 1-9 or a method according to any of claims 10-14 to assemble an elastic laminate (12) and/or to manufacture an absorbent article.

## Patentansprüche

1. Einrichtung (10) zum Dehnen einer Vielzahl von elastischen Streifen (16, 18) in einer Maschinenquerrichtung (CD), die Einrichtung (10) umfassend:
- einen ersten Satz von drehbaren Scheiben (34) zum Dehnen eines ersten elastischen Streifens (16) in einer Maschinenquerrichtung (CD), und
- einen zweiten Satz von drehbaren Scheiben (62) zum Dehnen eines zweiten elastischen Streifens (18) in einer Maschinenquerrichtung (CD), wobei:
eine erste drehbare Scheibe (36) jedes Satzes von drehbaren Scheiben (34, 62) in einem Winkel in Bezug auf eine zweite drehbare Scheibe (40) jedes Satzes von drehbaren Scheiben (34, 62) geneigt ist, und
∘ jede drehbare Scheibe (36, 40) eine Außenoberfläche (36e, 40e) aufweist, die mit mindestens einem Greifelement (50) bereitgestellt ist, das konfiguriert ist, um eine Kante eines elastischen Streifens (16, 18) an einem Aufgreifort aufzunehmen und die Kante des elastischen Streifens (16, 18) zu halten, wenn der elastische Streifen (16, 18) während des Betriebs in der Maschinenrichtung (MD) von dem Aufgreifort zu einem Abgabeort vorwärtsbewegt wird, wobei der Abstand zwischen der ersten drehbaren Scheibe (36) und der zweiten drehbaren Scheibe (40) jedes Satzes von drehbaren Scheiben (34, 62) an dem Abgabeort größer ist als an dem Aufgreifort, wodurch der elastische Streifen (16, 18) in der Maschinenquerrichtung (CD) gedehnt wird,
wobei die Einrichtung (10) Folgendes umfasst:
- eine gemeinsame Energiequelle (54),
- eine gemeinsame Antriebswelle (56), und
- einen Drehungsübertragungsmechanismus (58),
wobei die gemeinsame Antriebswelle (56) und der Drehungsübertragungsmechanismus (58) konfiguriert sind, um Energie von der gemeinsamen Energiequelle (54) sowohl auf den ersten Satz von drehbaren Scheiben (34) als auch auf den zweiten Satz von drehbaren Scheiben (62) zu übertragen, um die erste drehbare Scheibe (36) und die zweite drehbare Scheibe (40) jedes Satzes von drehbaren Scheiben (34, 62) zu drehen.

2. Einrichtung (10) nach Anspruch 1, wobei der Drehungsübertragungsmechanismus (58) mindestens eines der Folgenden oder eine Kombination davon umfasst: mindestens einen Riemen, mindestens einen Keilriemen, mindestens eine Kette, mindestens ein Zahnrad, mindestens eine direkte Verknüpfung zwischen der gemeinsamen Antriebswelle (56) und einem Satz von drehbaren Scheiben (34, 62), mindestens einen Steuerriemen, mindestens eine Steuerkette, mindestens ein Seil, mindestens ein Kabel, mindestens eine Riemenscheibe, mindestens ein Kettenrad, ein hydraulisches System, ein pneumatisches System.

3. Einrichtung (10) nach Anspruch 1 oder Anspruch 2, wobei eine erste drehbare Scheibe (36) von mindestens einem Satz von drehbaren Scheiben (34, 62) konfiguriert ist, um sich um eine erste Drehachse (38) zu drehen, und eine zweite drehbare Scheibe (40) des mindestens einen Satzes von drehbaren Scheiben (34, 62) konfiguriert ist, um sich um eine zweite Drehachse (42) zu drehen, wobei die erste Drehachse (38) in einem Winkel in Bezug auf die zweite Drehachse (42) geneigt ist

4. Einrichtung (10) nach einem der Ansprüche 1 bis 3, wobei die Einrichtung (10) eine Stützstruktur (60) umfasst und mindestens eine der drehbaren Scheiben (36, 40) verschiebbar auf der Stützstruktur (60) montiert ist.

5. Einrichtung (10) nach Anspruch 4, wobei die Stützstruktur (60) konfiguriert ist, um zu erlauben, dass mindestens einer der folgenden Parameter durch Verschieben der mindestens einen drehbaren Scheibe (36, 40) auf der Stützstruktur (60) eingestellt wird:
- der minimale Abstand (46) und/oder der maximale Abstand (48) zwischen der ersten drehbaren Scheibe (36) und der zweiten drehbaren Scheibe (40) eines Satzes von drehbaren Scheiben (34, 62),
- ein Abstand zwischen dem ersten Satz von drehbaren Scheiben (34) und dem zweiten Satz von drehbaren Scheiben (62),
- die Neigung der ersten drehbaren Scheibe (36) oder der ersten Drehachse (38),
- die Neigung der zweiten drehbaren Scheibe (40) oder der zweiten Drehachse (42).

6. Einrichtung (10) nach Anspruch 4 oder Anspruch 5, wobei die Stützstruktur (60) eine gemeinsame Stützstruktur ist und alle drehbaren Scheiben (36, 40) verschiebbar auf der gemeinsamen Stützstruktur (60) montiert sind.

7. Einrichtung (10) nach einem der Ansprüche 3 bis 6, wobei die Stützstruktur (60) mindestens einen drehbaren Scheibenpositionsanzeiger (64) umfasst, der konfiguriert ist, um eine Position der mindestens einen drehbaren Scheibe (36, 40) anzuzeigen, die verschiebbar daran montiert ist.

8. Einrichtung (10) nach einem der vorstehenden Ansprüche, wobei die Einrichtung (10) mindestens einen zusätzlichen Satz von drehbaren Scheiben zum Dehnen mindestens eines zusätzlichen elastischen Streifens in einer Maschinenquerrichtung (CD) umfasst, und die gemeinsame Antriebswelle (56) und der Drehungsübertragungsmechanismus konfiguriert sind, um Energie von der gemeinsamen Energiequelle (54) auf alle Sätze von drehbaren Scheiben zu übertragen, um die erste drehbare Scheibe (36) und die zweite drehbare Scheibe (40) jedes Satzes von drehbaren Scheiben zu drehen.

9. Einrichtung (10) nach einem der vorstehenden Ansprüche, wobei die Einrichtung (10) Folgendes umfasst:
- mindestens eine Zuführeinheit (14), die konfiguriert ist, um einen ersten elastischen Streifen (16) dem ersten Satz von drehbaren Scheiben (34) und einen zweiten elastischen Streifen (18) dem zweiten Satz von drehbaren Scheiben (62) zuzuführen,
- mindestens eine Zuführeinheit (22, 26), die konfiguriert ist, um mindestens eine Trägerbahn (24, 28) zuzuführen, und
- mindestens eine Verbindungsvorrichtung (30), die konfiguriert ist, um einen gedehnten ersten elastischen Streifen (16) und einen gedehnten zweiten elastischen Streifen (18) mit der mindestens einen Trägerbahn (24, 28) zu verbinden.

10. Verfahren zum Dehnen einer Vielzahl von elastischen Streifen (16, 18) in einer Maschinenquerrichtung (CD), das Verfahren umfassend:
- Zuführen eines ersten elastischen Streifens (16) zu einem ersten Satz von drehbaren Scheiben (34) zum Dehnen eines ersten elastischen Streifens (16) in einer Maschinenquerrichtung (CD),
- Zuführen eines zweiten elastischen Streifens (18) zu einem zweiten Satz von drehbaren Scheiben (62) zum Dehnen eines zweiten elastischen Streifens (18) in einer Maschinenquerrichtung (CD),
- Drehen einer ersten drehbaren Scheibe (36) jedes Satzes von drehbaren Scheiben (34, 62), und
- Drehen einer zweiten drehbaren Scheibe (40) jedes Satzes von drehbaren Scheiben (34, 62),
wobei die erste drehbare Scheibe (36) in einem Winkel in Bezug auf die zweite drehbare Scheibe geneigt ist, und
- Aufnehmen einer Kante eines elastischen Streifens (16, 18) auf einer Außenoberfläche (36e, 40e) einer ersten drehbaren Scheibe (36) jedes Satzes von drehbaren Scheiben und einer Kante des elastischen Streifens (16, 18) auf einer Außenoberfläche (36e, 40e) einer zweiten drehbaren Scheibe (40) jedes Satzes von drehbaren Scheiben an einem Aufgreifort,
- Halten des elastischen Streifens (16, 18) auf der Außenoberfläche (36e, 40e) jeder drehbaren Scheibe (36, 40) unter Verwendung mindestens eines Greifelements (50), und
- Vorwärtsbewegen des elastischen Streifens (16, 18) in der Maschinenrichtung (MD) von dem Aufgreifort zu einem Abgabeort, wobei der Abstand zwischen der ersten drehbaren Scheibe (36) und der zweiten drehbaren Scheibe (40) jedes Satzes von drehbaren Scheiben (34, 62) an dem Abgabeort größer ist als an dem Aufgreifort, wodurch der elastische Streifen (16, 18) in der Maschinenquerrichtung (CD) gedehnt wird,
wobei das Verfahren Folgendes umfasst:
- Drehen der ersten drehbaren Scheibe (36) und der zweiten drehbaren Scheibe (40) jedes Satzes von drehbaren Scheiben (34, 62) durch Übertragen von Energie von einer gemeinsamen Energiequelle (54) sowohl auf den ersten Satz von drehbaren Scheiben (34) als auch auf den zweiten Satz von drehbaren Scheiben (62) über eine gemeinsame Antriebswelle (56) und einen Drehungsübertragungsmechanismus (58).

11. Verfahren nach Anspruch 10, wobei das Verfahren das Bereitstellen eines Drehungsübertragungsmechanismus (58) umfasst, der mindestens eines der Folgenden oder eine Kombination davon umfasst: mindestens einen Riemen, mindestens einen Keilriemen, mindestens eine Kette, mindestens ein Zahnrad, mindestens eine direkte Verknüpfung zwischen der gemeinsamen Energiequelle (54) und der gemeinsamen Antriebswelle (56), mindestens einen Steuerriemen, mindestens eine Steuerkette, mindestens ein Seil, mindestens ein Kabel, mindestens eine Riemenscheibe, mindestens ein Kettenrad, ein hydraulisches System, ein pneumatisches System.

12. Verfahren nach Anspruch 10 oder Anspruch 11, wobei das Verfahren das verschiebbare Montieren mindestens einer drehbaren Scheibe (36, 40) des mindestens einen Satzes von drehbaren Scheiben (34, 62) an einer Stützstruktur (60) und das Einstellen mindestens eines der folgenden Parameter durch Verschieben der mindestens einen drehbaren Scheibe (36, 40) auf der Stützstruktur (60) umfasst:
- der minimale Abstand (46) und/oder der maximale Abstand (48) zwischen der ersten drehbaren Scheibe (36) und der zweiten drehbaren Scheibe (40) eines Satzes von drehbaren Scheiben (34, 62),
- ein Abstand zwischen dem ersten Satz von drehbaren Scheiben (34) und dem zweiten Satz von drehbaren Scheiben (62),
- die Neigung der ersten drehbaren Scheibe (36) oder der ersten Drehachse (38),
- die Neigung der zweiten drehbaren Scheibe (40) oder der zweiten Drehachse (42).

13. Verfahren nach Anspruch 12, wobei das Verfahren das Bereitstellen einer gemeinsamen Stützstruktur (60) und das verschiebbare Montieren aller drehbaren Scheiben (36, 40) aller Sätze von drehbaren Scheiben (34, 62) auf der gemeinsamen Stützstruktur (60) umfasst.

14. Verfahren nach einem der Ansprüche 10 bis 13, wobei das Verfahren Folgendes umfasst:
- Zuführen eines ersten elastischen Streifens (16) zu dem ersten Satz von drehbaren Scheiben (34),
- Zuführen eines zweiten elastischen Streifens (18) zu dem zweiten Satz von drehbaren Scheiben (62),
- Zuführen mindestens einer Trägerbahn (24, 28), und
- Verbinden des ersten elastischen Streifens (16) und des zweiten elastischen Streifens (18) mit der mindestens einen Trägerbahn (24, 28).

15. Verwendung einer Einrichtung (10) nach einem der Ansprüche 1 bis 9 oder eines Verfahrens nach einem der Ansprüche 10 bis 14, um ein elastisches Laminat (12) zusammenzuführen und/oder einen saugfähigen Artikel herzustellen.

## Revendications

1. Appareil (10) permettant d'étirer une pluralité de bandes élastiques (16, 18) dans un sens transversal de la machine (CD), l'appareil (10) comprenant :
- un premier ensemble de disques rotatifs (34) permettant d'étirer une première bande élastique (16) dans un sens transversal de la machine (CD), et
- un second ensemble de disques rotatifs (62) permettant d'étirer une seconde bande élastique (18) dans un sens transversal de la machine (CD), moyennant quoi :
un premier disque rotatif (36) de chaque ensemble de disques rotatifs (34, 62) est incliné à un angle par rapport à un second disque rotatif (40) de chaque ensemble de disques rotatifs (34, 62), et
∘ chaque disque rotatif (36, 40) a une surface externe (36e, 40e) pourvue d'au moins un élément de préhension (50) conçu pour recevoir un bord d'une bande élastique (16, 18) au niveau d'un emplacement de collecte et maintenir le bord de la bande élastique (16, 18) lorsque la bande élastique (16, 18) est avancée dans le sens machine (MD) de l'emplacement de collecte à un emplacement de dépôt pendant le fonctionnement, moyennant quoi la distance entre le premier disque rotatif (36) et le second disque rotatif (40) de chaque ensemble de disques rotatifs (34, 62) est plus grande au niveau de l'emplacement de dépôt qu'au niveau de l'emplacement de collecte, étirant de ce fait la bande élastique (16, 18) dans le sens transversal de la machine (CD),
dans lequel l'appareil (10) comprend :
- une source d'alimentation commune (54),
- un arbre d'entraînement commun (56), et
- un mécanisme de transmission de rotation (58),
moyennant quoi l'arbre d'entraînement commun (56) et le mécanisme de transmission de rotation (58) sont conçus pour transmettre l'énergie de la source d'alimentation commune (54) à la fois au premier ensemble de disques rotatifs (34) et au second ensemble de disques rotatifs (62) pour mettre en rotation le premier disque rotatif (36) et le second disque rotatif (40) de chaque ensemble de disques rotatifs (34, 62).

2. Appareil (10) selon la revendication 1, dans lequel le mécanisme de transmission de rotation (58) comprend au moins un ou une combinaison des éléments suivants : au moins une courroie, au moins une courroie trapézoïdale, au moins une chaîne, au moins un engrenage, au moins une liaison directe entre l'arbre d'entraînement commun (56) et un ensemble de disques rotatifs (34, 62), au moins une courroie de distribution, au moins une chaîne de distribution, au moins une corde, au moins un câble, au moins une poulie, au moins un pignon, un système hydraulique, un système pneumatique.

3. Appareil (10) selon la revendication 1 ou la revendication 2, dans lequel un premier disque rotatif (36) d'au moins un ensemble de disques rotatifs (34, 62) est conçu pour tourner autour d'un premier axe de rotation (38), et un second disque rotatif (40) de l'au moins un ensemble de disques rotatifs (34, 62) est conçu pour tourner autour d'un second axe de rotation (42), moyennant quoi le premier axe de rotation (38) est incliné à un angle par rapport au second axe de rotation (42)

4. Appareil (10) selon l'une quelconque des revendications 1 à 3, dans lequel l'appareil (10) comprend une structure de support (60) et au moins l'un des disques rotatifs (36, 40) est monté de manière coulissante sur la structure de support (60).

5. Appareil (10) selon la revendication 4, dans lequel la structure de support (60) est conçue pour permettre le réglage d'au moins l'un parmi les paramètres suivants en faisant coulisser l'au moins un disque rotatif (36, 40) sur la structure de support (60) :
- la distance minimale (46) et/ou la distance maximale (48) entre le premier disque rotatif (36) et le second disque rotatif (40) d'un ensemble de disques rotatifs (34, 62),
- un espacement entre le premier ensemble de disques rotatifs (34) et le second ensemble de disques rotatifs (62),
- l'inclinaison du premier disque rotatif (36) ou du premier axe de rotation (38),
- l'inclinaison du second disque rotatif (40) ou du second axe de rotation (42).

6. Appareil (10) selon la revendication 4 ou la revendication 5, dans lequel la structure de support (60) est une structure de support commune, et tous les disques rotatifs (36, 40) sont montés de manière coulissante sur la structure de support commune (60).

7. Appareil (10) selon l'une quelconque des revendications 3 à 6, dans lequel la structure de support (60) comprend au moins un indicateur de position de disque rotatif (64) conçu pour indiquer une position de l'au moins un disque rotatif (36, 40) qui est monté de manière coulissante sur celui-ci.

8. Appareil (10) selon l'une quelconque revendication précédente, dans lequel l'appareil (10) comprend au moins un ensemble supplémentaire de disques rotatifs permettant d'étirer au moins une bande élastique supplémentaire dans un sens transversal de la machine (CD), et l'arbre d'entraînement commun (56) et le mécanisme de transmission de rotation sont conçus pour transmettre l'énergie de la source d'alimentation commune (54) à tous les ensembles de disques rotatifs pour mettre en rotation le premier disque rotatif (36) et le second disque rotatif (40) de chaque ensemble de disques rotatifs.

9. Appareil (10) selon l'une quelconque revendication précédente, dans lequel l'appareil (10) comprend :
- au moins une unité d'alimentation (14) conçue pour fournir une première bande élastique (16) au premier ensemble de disques rotatifs (34) et une seconde bande élastique (18) au second ensemble de disques rotatifs (62),
- au moins une unité d'alimentation (22, 26) conçue pour alimenter au moins une bande de support (24, 28), et
- au moins un dispositif de liaison (30) conçu pour lier une première bande élastique étirée (16) et une seconde bande élastique étirée (18) à l'au moins une bande de support (24, 28).

10. Procédé permettant d'étirer une pluralité de bandes élastiques (16, 18) dans un sens transversal de la machine (CD), le procédé comprenant :
- la fourniture d'une première bande élastique (16) à un premier ensemble de disques rotatifs (34) permettant d'étirer une première bande élastique (16) dans un sens transversal de la machine (CD),
- la fourniture d'une seconde bande élastique (18) à un second ensemble de disques rotatifs (62) permettant d'étirer une seconde bande élastique (18) dans un sens transversal de la machine (CD),
- la mise en rotation d'un premier disque rotatif (36) de chaque ensemble de disques rotatifs (34, 62), et
- la mise en rotation d'un second disque rotatif (40) de chaque ensemble de disques rotatifs (34, 62),
moyennant quoi le premier disque rotatif (36) est incliné à un angle par rapport au second disque rotatif, et
- la réception d'un bord d'une bande élastique (16, 18) sur une surface externe (36e, 40e) d'un premier disque rotatif (36) de chaque ensemble de disques rotatifs et d'un bord de la bande élastique (16, 18) sur une surface externe (36e, 40e) d'un second disque rotatif (40) de chaque ensemble de disques rotatifs au niveau d'un emplacement de collecte,
- le maintien de la bande élastique (16, 18) sur la surface externe (36e, 40e) de chaque disque rotatif (36, 40) à l'aide d'au moins un élément de préhension (50), et
- l'avancement de la bande élastique (16, 18) dans le sens machine (MD) de l'emplacement de collecte à un emplacement de dépôt, moyennant quoi la distance entre le premier disque rotatif (36) et le second disque rotatif (40) de chaque ensemble de disques rotatifs (34, 62) est plus grande au niveau de l'emplacement de dépôt qu'au niveau de l'emplacement de collecte, étirant de ce fait la bande élastique (16, 18) dans le sens transversal de la machine (CD),
le procédé comprenant :
- la rotation du premier disque rotatif (36) et du second disque rotatif (40) de chaque ensemble de disques rotatifs (34, 62) en transmettant de l'énergie d'une source d'alimentation commune (54) à la fois au premier ensemble de disques rotatifs (34) et au second ensemble de disques rotatifs (62) par l'intermédiaire d'un arbre d'entraînement commun (56) et d'un mécanisme de transmission de rotation (58),

11. Procédé selon la revendication 10, dans lequel le procédé comprend la fourniture d'un mécanisme de transmission de rotation (58) qui comprend au moins un ou une combinaison des éléments suivants : au moins une courroie, au moins une courroie trapézoïdale, au moins une chaîne, au moins un engrenage, au moins une liaison directe entre la source d'alimentation commune (54) et un arbre d'entraînement commun (56), au moins une courroie de distribution, au moins une chaîne de distribution, au moins une corde, au moins un câble, au moins une poulie, au moins un pignon, un système hydraulique, un système pneumatique.

12. Procédé selon la revendication 10 ou la revendication 11, dans lequel le procédé comprend le montage coulissant d'au moins un disque rotatif (36, 40) de l'au moins un ensemble de disques rotatifs (34, 62) sur une structure de support (60), et le réglage d'au moins l'un parmi les paramètres suivants en faisant coulisser l'au moins un disque rotatif (36, 40) sur la structure de support (60) :
- la distance minimale (46) et/ou la distance maximale (48) entre le premier disque rotatif (36) et le second disque rotatif (40) d'un ensemble de disques rotatifs (34, 62),
- un espacement entre le premier ensemble de disques rotatifs (34) et le second ensemble de disques rotatifs (62),
- l'inclinaison du premier disque rotatif (36) ou du premier axe de rotation (38),
- l'inclinaison du second disque rotatif (40) ou du second axe de rotation (42).

13. Procédé selon la revendication 12, dans lequel le procédé comprend la fourniture d'une structure de support commune (60) et le montage coulissant de tous les disques rotatifs (36, 40) de tous les ensembles de disques rotatifs (34, 62) sur la structure de support commune (60).

14. Procédé selon l'une quelconque des revendications 10 à 13, dans lequel le procédé comprend :
- la fourniture d'une première bande élastique (16) au premier ensemble de disques rotatifs (34),
- la fourniture d'une seconde bande élastique (18) au second ensemble de disques rotatifs (62),
- la fourniture d'au moins une bande de support (24, 28), et
- la liaison de la première bande élastique (16) et de la seconde bande élastique (18) à l'au moins une bande de support (24, 28).

15. Utilisation d'un appareil (10) selon l'une quelconque des revendications 1 à 9 ou d'un procédé selon l'une quelconque des revendications 10 à 14 pour assembler un stratifié élastique (12) et/ou pour fabriquer un article absorbant.
